# FASCICULE DE BREVET EUROPEEN

(11) **EP 3 087 551 B1**
(45) Date de publication et mention de la délivrance du brevet: **15.08.2018**
(21) Numéro de dépôt: 14825353.7
(22) Date de dépôt: 23.12.2014
(51) Int. Cl.: G06T 7/00, A61B 5/00

(54) **PROCÉDÉ D'ANALYSE D'IMAGES DU VISAGE POUR LA DÉTECTION D'UN EFFET FLUSH**
VERFAHREN ZUR ANALYSE VON GESICHTSBILDERN ZUR DETEKTION EINES ERKENNUNG EINES FLUSH-EFFEKTS
METHOD OF ANALYSING FACIAL IMAGES FOR DETECTING A FLUSH EFFECT

(30) Priorité: 23.12.2013 FR 1363400
(43) Date de publication de la demande: 02.11.2016
(73) Titulaire: Galderma Research & Development, 06410 Biot (FR)
(72) Inventeur: PETIT, Laurent, F-06530 Peymeinade (FR)
(74) Mandataire: Casalonga
(86) Numéro de dépôt international: PCT/EP2014/079227
(87) Numéro de publication internationale: WO 2015/097239

(56) Documents cités:
- WO-A1-2013/122233
- JP-A- 2007 152 084
- JENNIFER K. WAGNER ET AL: "Comparing Quantitative Measures of Erythema, Pigmentation and Skin Response using Reflectometry", PIGMENT CELL RESEARCH, vol. 15, no. 5, 1 octobre 2002 (2002-10-01), pages 379-384, XP055124308, ISSN: 0893-5785, DOI: 10.1034/j.1600-0749.2002.02042.x
- H Takiwaki: "Measurement of skin color: practical application and theoretical considerations", The journal of medical investigation : JMI, vol. 44 1 février 1998 (1998-02-01), page 121, XP055124277, JAPAN Extrait de l'Internet: URL:http://www.ncbi.nlm.nih.gov/pubmed/959 7799 [extrait le 2014-06-19]
- _ A FULLERTON ET AL: "Guidelines for measurement of skin colour and erythema - A report from the Standardization Group of the European Society of Contact Dermatitisi", CONTACT DERMATITIS, vol. 35, no. 1, 1 janvier 1996 (1996-01-01), pages 1-10, XP055138222,
- Xiaochao Yang ET AL: "Visage: A Face Interpretation Engine for Smartphone Applications" In: "Sensor Applications, Experimentation, and Logistics", 1 janvier 2013 (2013-01-01), Springer Berlin Heidelberg, Berlin, Heidelberg, XP055171510, ISSN: 1867-8211 ISBN: 978-3-64-211870-8 vol. 110, pages 149-168, DOI: 10.1007/978-3-642-36632-1_9, abrégé section 3.1 figures 1,4,5

## Description

L'invention a pour domaine technique l'analyse de peau, et plus particulièrement l'analyse de l'évolution des paramètres colorimétriques de la peau.

Dans ce domaine, est connu le brevet japonais JP 2007-152084 qui décrit un procédé d'analyse de l'évolution de la couleur de la peau et de comparaison de la couleur d'un sujet à des valeurs moyennes de la population.

L'invention a pour objet un procédé d'analyse d'images du visage d'un être humain tel que définit par la revendication 1.

Les zones du visage peuvent être les joues.

Les composantes peuvent être les composantes colorimétriques L* (clarté), a* (axe rouge/vert), b* (axe jaune/bleu).

Les valeurs mémorisées peuvent être les valeurs des composantes colorimétriques déterminées à une date antérieure pour le même visage.

On peut réaliser l'acquisition d'images par l'intermédiaire d'un téléphone intelligent ou ordiphone.

Par ailleurs, la verticalité de l'acquisition d'images peut être assurée par un capteur interne d'un téléphone intelligent, par exemple un gyroscope à trois axes.

On peut réaliser l'acquisition alors que le sujet tient une mire colorimétrique par l'intermédiaire de la bouche, afin de corriger les composantes colorimétriques.

On peut acquérir au moins un paramètre choisi parmi la température, la pression atmosphérique, la luminosité ambiante et l'hygrométrie, afin de corriger les composantes colorimétriques d'un effet de l'environnement sur le visage.

Avantageusement, les valeurs des composantes colorimétriques déterminées peuvent être configurées pour une auto-évaluation des maladies de la peau tels que le mélasma, le nævus, la rosacée, l'érythème, le rougissement, l'acné, le psoriasis, la dermatite, la kératose actinique, l'éruption cutanée et la dermite séborrhéique.

D'autres buts, caractéristiques et avantages de l'invention apparaîtront à la lecture de la description suivante, donnée uniquement à titre d'exemple non limitatif et faite en référence aux dessins annexés sur lesquels :
- les figures 1a et 1b illustrent des exemples de photographies acquises avec deux systèmes d'acquisition différents,
- les figures 2a, 2b, 2c, 3a et 3b illustrent le fonctionnement d'un logiciel d'acquisition,
- les figures 4a, 4b et 4c illustrent des mires colorimétriques,
- les figures 5a et 5b illustrent deux vues d'un support de mire colorimétrique,
- la figure 6 illustre les zones analysées sur une photographie,
- les figures 7, 8 et 9 illustrent respectivement l'évolution de des paramètres colorimétrique L*, a* et b* en fonction du temps, sur 20 jours,
- les figures 10, 11 et 12 illustrent l'évolution de chaque paramètre colorimétrique pour la moyenne des deux joues d'un individu, par jour,
- les figures 13 à 15 illustrent l'évolution de l'ensemble des paramètres colorimétriques indépendamment pour chaque joue,
- les figures 16 à 18 illustrent l'évolution colorimétrique globale du visage (joue droite et joue gauche confondues),
- les figures 19a, 19b, 20a et 20b illustrent l'évolution de la couleur de la peau sous l'effet du bronzage,
- les figures 21, 22 et 23 illustrent l'évolution colorimétrique de chaque joue,
- les figures 24, 25 et 26 illustrent l'évolution colorimétrique globale,
- les figures 28a et 28b illustrent des photos prises avec un niveau de luminosité ambiante très important,
- la figure 29 illustre l'évolution du taux d'hygrométrie au cours du temps,
- la figure 30 illustre l'évolution de la température au cours du temps,
- la figure 31 illustre l'évolution de la pression atmosphérique au cours du temps,
- la figure 32 illustre les différences de luminosité ambiante observées sur les acquisitions d'une autre personne,
- la figure 33 illustre le taux d'hygrométrie en fonction de temps sur les acquisitions d'une autre personne,
- la figure 34 illustre la température en fonction de temps sur les acquisitions d'une autre personne,
- la figure 35 illustre l'évolution de la pression atmosphérique au cours du temps sur les acquisitions d'une autre personne,
- la figure 36 illustre l'évolution de la luminosité ambiante,
- la figure 37 illustre des photos acquises avec une luminosité ambiante dépassant les 4000 lux,
- les figures 38a, 38b, 39a et 39b illustrent l'influence de l'inclinaison verticale lors de l'acquisition d'une photographie,
- les figures 40a et 40b illustrent deux objectifs déportés et connectés ;
- les figures 41a et 41b illustrent deux phases d'acquisition de données pour le test d'une application logicielle d'acquisition de données"
- les figures 42a et 42b sont deux photos d'un sujet prises lors des phases des figures 41a et 41b, respectivement le matin et lors d'un effet flush, et
- la figure 43 illustre l'évolution du nombre de flushs pendant la journée enregistrée par le logiciel d'acquisition selon l'invention.

On décrira en premier lieu un protocole technique permettant l'analyse de l'effet flush ou rougissement brutal sur un visage. Le but est de quantifier la modification de couleur du visage, qui devient plus rouge, pendant cet effet flush, la finalité étant de conduire une étude clinique pour montrer la diminution de ces effets avec la prise d'un principe actif spécifique. Avantageusement, cette étude peut être utilisée pour une auto-évaluation des maladies de la peau tels que le mélasma, le nævus, la rosacée, l'érythème, le rougissement, l'acné, le psoriasis, la dermatite, la kératose actinique, l'éruption cutanée et la dermite séborrhéique.

Une première étude a permis de définir un système portable le plus optimal pour l'acquisition de photos en mode portrait. Il a été décidé d'utiliser deux systèmes de prise de vues distincts, a savoir un premier et un deuxième appareils respectivement commercialisés par la société Samsung sous les marques :
- Samsung Galaxy S4 ®
- Samsung Galaxy S4 Mini ®

Le but est de caractériser les deux systèmes d'acquisition choisis et de valider la sensibilité d'acquisition en fonction des conditions d'illumination et de l'environnement de la prise de vue.

Après une description des spécifications des deux systèmes et du logiciel d'acquisition désigné par la suite par le terme de « SmartCam » développé spécifiquement pour cette application, le protocole d'acquisition et les méthodes d'analyse seront détaillés.

Les résultats, les problèmes rencontrés et les solutions et préconisations envisagées seront ensuite détaillées.

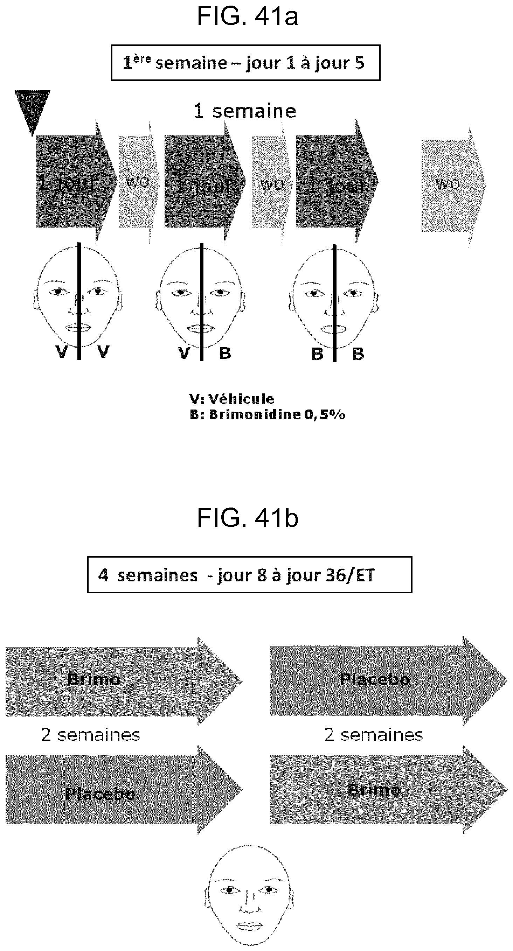

Une utilisation pratique de l'application logicielle embarquée dans les systèmes de prise de vues sera enfin décrite en référence aux figures 41a, 41b, 42a, 42b et 43 dans le cadre d'un traitement d'un flush par le Brimonidine.

Pour effectuer l'ensemble des acquisitions, comme indiqué précédemment, deux smartphones ou téléphone intelligent, en langue française, à savoir le téléphone commercialisé sous la marque Samsung Galaxy S4, ainsi qu'un téléphone Samsung Galaxy S4 Mini qui a un encombrement plus réduit mais des performances un peu plus faibles que le Samsung Galaxy S4. Avec le deuxième téléphone S4 Mini, la résolution de l'appareil photo est légèrement plus faible et l'ensemble des capteurs d'humidité, température, hygrométrie ne sont pas présents.

Les caractéristiques du premier appareil (Samsung Galaxy S4) sont les suivantes :

| | |
|---|---|
| Dimensions | 136,6 x 69,8 x 7,9 mm |
| Poids | 130 g |
| Résolution caméra frontale | 2,1 M Pixel (1920x1080) |
| Résolution caméra dorsale | 13 M Pixels |
| Système d'exploitation | Android |
| Téléphone | Oui |
| GPS | Oui |
| Capteurs | Proximité, Lumière, ambiante, Thermomètre, Hygromètre, Baromètre, Accéléromètre, Gyroscope à trois axes, Magnétomètre |

Les caractéristiques du deuxième appareil (Samsung® Galaxy S4 Mini) sont les suivantes :

| | |
|---|---|
| Dimensions | 124,6 x 61,3 x 8,9 mm |
| Poids | 107g |
| Résolution caméra frontale | 1,9 M Pixels (1392x1392) |
| Résolution caméra dorsale | 8 M Pixels |
| Système d'exploitation | Android |
| Téléphone | Oui |
| GPS | Oui |
| Capteurs | Proximité, Accéléromètre, Gyroscope à trois axes, Magnétomètre |

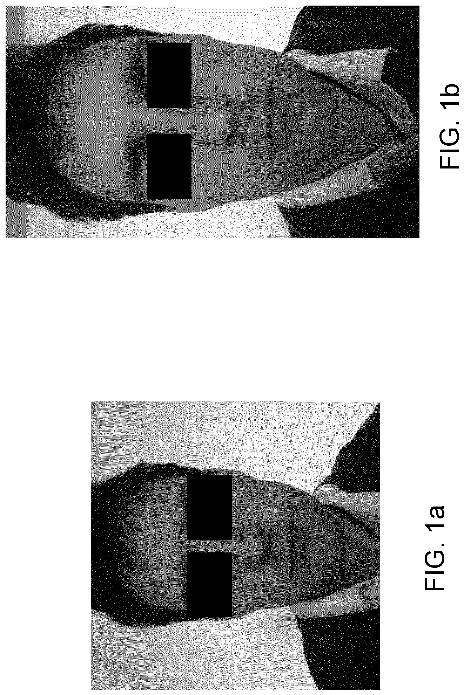

Afin de comparer les deux systèmes, une série de photos ont été prises dans les mêmes conditions d'éclairage et à quelques secondes d'intervalles avec le Samsung Galaxy S4 et le Samsung Galaxy S4 Mini. Les figures 1a et 1b présentent des exemples de photos acquises avec les deux systèmes, la figure la étant acquise avec le Samsung Galaxy S4 Mini (1393x1393) et la figure 1b étant acquise avec le Samsung Galaxy S4 (1080x1920).

On observe alors que la résolution du Galaxy S4 Mini est carrée (1393x1393 pixels) et nécessite de se positionner un peu plus loin de l'objectif par rapport au Galaxy S4 (1080x1920 pixels).

Le fait de se positionner plus loin de l'objectif entraîne une diminution des distorsions optiques (communément désigné par l'homme du métier sous l'acronyme anglo-saxon « fish-eye effects ») qui sont légèrement visibles avec le Samsung® Galaxy S4. L'analyse des photos étant axée sur des analyses non morphologiques, cela n'a pas d'effets négatifs. Par contre, on note que dans le même environnement d'acquisition, le Galaxy S4 prend des photos plus lumineuse et que le brillant est lui aussi plus nettement visible sur la peau. Les images sont également plus contrastées avec le Galaxy S4 en comparaison de celles acquises avec le Samsung Galaxy S4 Mini.

Et les détails sont aussi plus visibles avec le Galaxy S4 que sur le Galaxy S4 Mini.

Par ailleurs, un logiciel d'acquisition de données, embarqué dans les téléphones permet notamment :
- de définir, au début de l'étude, deux axes horizontaux sur la photo en mode « live » (temps réel, en langue française) permettant d'aligner les yeux et la bouche du sujet,
- de permettre le repositionnement rapide du sujet à l'aide de ces deux axes horizontaux et d'un axe vertical,
- de définir un indicateur d'inclinaison verticale permettant de limiter la variabilité d'angle d'acquisition d'images,
- de stocker les images acquises en haute définition (résolution maximale de l'appareil) au format JPEG, et
- de stocker les données relatives à chaque prise d'image comme la date et l'heure mais aussi les informations des différents capteurs : luminosité ambiante, pression atmosphérique, température, hygrométrie et inclinaison verticale.

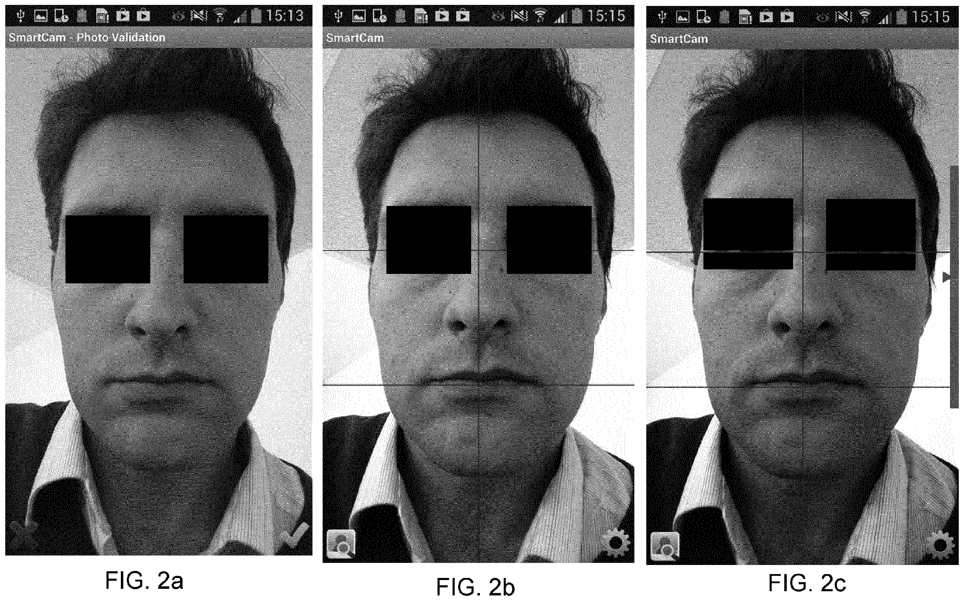

Au lancement du logiciel, le mode « live » s'affiche directement avec les axes horizontaux et l'axe verticale (figure 2a). Ces axes ont été définis dans la zone d'administration du logiciel au début de l'étude.

L'utilisateur se positionne devant l'appareil photo et ajuste au mieux son visage pour s'aligner sur les repères.

Une fois le sujet bien positionné, celui-ci appuie sur l'écran pour prendre la photo. Une nouvelle fenêtre apparaît avec la photo acquise en pleine résolution et l'utilisateur peut choisir de la conserver ou de l'annuler (figure 2b).

L'application logicielle permet également d'afficher un indicateur d'inclinaison sur l'interface (au milieu à droite) du logiciel en mode « live » pour forcer le bon positionnement de l'appareil à la verticale (zone verte de l'indicateur d'inclinaison) au moment de la prise d'image (figure 2c).

Lorsque la photo est validée, celle-ci est enregistrée dans le dossier « Pictures/SmartCam » de l'appareil et les données relatives sont stockées dans la base de données.

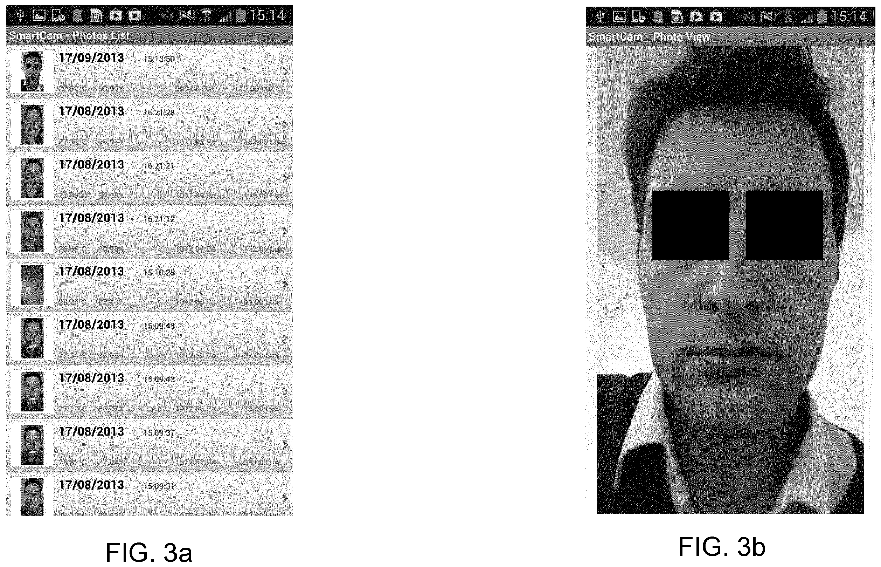

L'ensemble de ces données sont consultables par l'utilisateur (figure 3a). On retrouve alors la liste de toutes les photos acquises avec la date, l'heure, la luminosité ambiante et sur les appareils le permettant : la température, le taux d'humidité et la pression atmosphérique.

L'utilisateur peut aussi voir les photos en plein écran avec une interface dédiée (figure 3b) avec la possibilité de zoomer sur la photo pour voir les détails.

Dans la partie administration du logiciel, il est possible d'exporter l'ensemble des données stockées dans la base de données pour générer un fichier texte formaté pour être importé facilement dans Microsoft Excel ®.

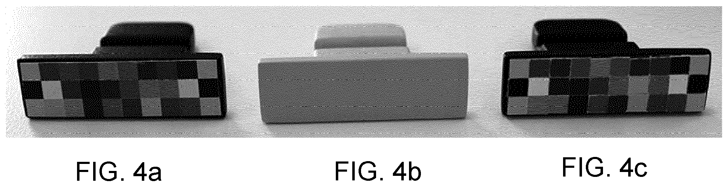

Afin de quantifier les dérives d'éclairement et de les corriger en fonction des différents environnements d'acquisition, trois mires spécifiques (figures 4a, 4b et 4c) sont utilisées pour mettre en oeuvre une étude de faisabilité :
- une mire numérique 30 patchs de couleur au format 40x12 mm avec des patchs de 4x4 mm (Figure 4a),
- une mire uniforme grise (Figure 4b), et
- une mire avec 30 patchs diffus Munsell au format 40x12 mm avec des patchs de 4x4 mm (Figure 4c).

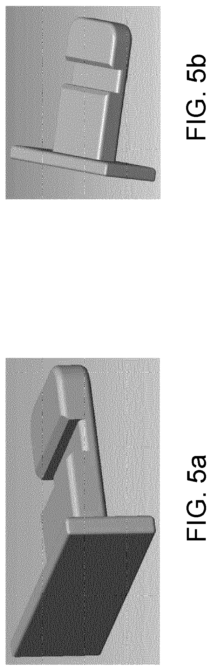

Un support spécifique en CAO (figures 5a et 5b) est également utilisé pour permettre à l'utilisateur de positionner la mire devant sa bouche de manière reproductible.

Le protocole d'acquisition des données doit permettre principalement:
- de valider la prise en main du logiciel d'acquisition « SmartCam »,
- de valider la qualité (résolution, rendu colorimétrique) des images résultantes,
- d'étudier les performances des différentes mires colorimétriques, et
- d'analyser les variabilités d'acquisition en fonction de l'environnement.

Le système d'acquisition doit être placé le plus verticalement possible devant le visage.

Le visage doit couvrir l'ensemble du champ d'acquisition pour maintenir une bonne résolution.

Lors de la première prise d'image, l'utilisateur doit définir les guides de repositionnement en déplaçant des curseurs rouge et vert sur les yeux et la bouche.

Il ne faut pas modifier les guides de repositionnement au cours de l'étude.

Il est possible néanmoins de déplacer les axes (Menu paramètre en bas à droite) au tout début de l'étude si ceux-ci ne sont pas correctement positionnés.

Une prise de vues s'effectue selon la procédure suivante :
- positionner la mire dans votre bouche en la maintenant par la rainure avec les dents
- sur l'écran d'accueil du logiciel d'acquisition « SmartCam » afficher : « positionnez-vous le mieux possible pour être centré par rapport à l'axe vertical et par rapport aux guides horizontaux de la bouche et des yeux »,
- cliquer n'importe où sur l'image pour prendre la photo
- une fenêtre apparaissant avec la photo acquise, la valider si celle-ci est correcte (pas de flou, bon positionnement) ou annuler pour recommencer.

Les photos acquises ne sont pas effaçables à partir du logiciel « SmartCam ».

Les photos acquises sont consultables en cliquant sur le bouton en bas à gauche de l'écran d'accueil. La liste de toutes les acquisitions apparaît et il est possible de cliquer sur un élément pour voir la photo en résolution complète à l'écran.

Il est nécessaire de bien s'assurer que la mire est présente sur chaque photo (sinon celle-ci sera inexploitable).

Il faudrait, au moins réaliser ces étapes au moins pendant 10 jours consécutifs. Les prises d'images suivantes doivent se faire dans des conditions normales (pas d'effort particulier avant) et si possible à heures fixes et dans le même environnement :
- 3 acquisitions de suite avec la mire le matin (lever)
- 3 acquisitions de suite le midi avec la mire le midi (si possible dans le même environnement que le matin)
- 3 acquisitions de suite le soir avec la mire le soir (coucher).

D'autres prises d'images peuvent être effectuées :
- au cours de la journée et dans différents lieux en extérieur et intérieur toujours avec la mire et toujours 3 fois de suite pour analyser la reproductibilité du système, et
- dans des conditions particulières (après le sport, après un temps d'exposition au soleil, ...)

Suite à l'ensemble des acquisitions réalisées les photos suivantes sont disponibles :
- 51 photos d'un premier individu prises avec un Galaxy S4 Mini réparties comme suit :
   ∘ 17 photos avec 3 répétitions soit 51 photos avec la mire numérique ;
- 168 photos d'un deuxième individu avec un Galaxy S4 réparties comme suit :
   ∘ 28 photos avec 3 répétitions soit 84 photos avec la mire numérique, et
   ∘ 28 photos avec 3 répétitions soit 84 photos avec la mire grise ;
- 450 photos prises avec un Galaxy S4 réparties comme suit:
   ∘ 50 photos avec 3 répétitions soit 150 photos avec la mire numérique,
   ∘ 50 photos avec 3 répétitions soit 150 photos avec la mire grise, et
   ∘ 50 photos avec 3 répétitions soit 150 photos avec la mire Munsell.

Soit un total de 669 photos à analyser.

Afin de définir quelle mire est la plus performante, les écarts relatifs des 3 composantes L*, a* et b* sont analysés sur un ensemble de 50 acquisitions avec 3 répétitions à chaque fois. Les photos ont été acquises avec le premier appareil, un Samsung Galaxy S4.

Chaque photo a été recalée colorimétriquement par des algorithmes de recalage colorimétrique avec ajustement des contraintes de convergence étant données les variations entre chaque condition de prise de vue qui sont très importantes.

Pour les mires 30 patchs, des algorithmes de recalage colorimétrique multi patchs sont utilisés alors que pour la mire grise, une technique de type « white point compensation » est utilisée.

Les résultats du recalage colorimétrique sont comparés avec chaque mire sur les composantes L*, a* et b* d'une zone définie sur chaque joue. L'écart type des valeurs moyennes des 2 joues (Tableau 1) est calculé sur les 3 répétitions après recalage colorimétrique.

**Tableau 1 : Ecart type relatif moyen des 2 joues avec 3 répétitions de 50 acquisitions pour chaque type de mire**

| | σ (L*) | σ (a*) | σ (b*) |
|---|---|---|---|
| Mire Numérique 30 patchs | 1,44 | 0,77 | 0,21 |
| Mire Grise | 1,65 | 1.20 | 1,37 |
| Mire Munsell 30 patchs | 1,46 | 0,95 | 1,04 |

On constate que les écarts relatifs (Tableau 1) entre 3 répétitions après correction colorimétrique sont du même ordre de grandeur quelle que soit la mire utilisée. Les variations observées semblent moins importantes avec la mire numérique 30 patchs. En effet, celle-ci présente un pelliculage légèrement brillant qui peut optimiser la correction colorimétrique.

On étudiera ci-dessous la performance de la correction colorimétrique (Tableau 2) sur l'ensemble des 30 patchs de la mire numérique sur les 150 photos acquises (50 photos avec 3 répétitions) avec cette mire.

**Tableau 2 : Variabilités moyennes des 30 patchs de la mire numérique avant et après correction colorimétrique sur 150 photos**

| | σ (L*) | σ (a*) | σ (b*) |
|---|---|---|---|
| Avant recalage | 6,62 | 6,75 | 9,32 |
| Après recalage | 2,64 | 3,61 | 5,14 |

On constate une diminution significative sur l'ensemble des paramètres colorimétriques L*, a* et b*.

On optimise de près de 50% la variabilité colorimétrique moyenne sur l'ensemble des teintes. Toutes les photos analysées dans le reste de cette étude seront celles qui sont recalées par rapport à la mire numérique 30 patchs.

Il sera maintenant décrit la reproductibilité de l'acquisition avec le recalage colorimétrique. Dix acquisitions successives de vues sont faites avec une mire colorimétrique numérique. Chaque acquisition est effectuée dans le même environnement mais la mire est retirée puis repositionnée entre chaque acquisition.

Les résultats présentés sur le tableau 3 suivant tiennent donc compte de la variabilité de repositionnement de la mire.

**Tableau 3 : Variabilités moyennes sur les paramètres colorimétriques des joues sur 10 photos prises successivement dans le même environnement.**

| | σ (L*) | σ (a*) | σ (b*) |
|---|---|---|---|
| Joue gauche | 1,20 | 0,73 | 0,16 |
| Joue droite | 1,19 | 0,52 | 0,14 |

On constate que les variabilités sont sensiblement identiques pour la joue droite et la joue gauche. La variabilité est plus importante sur la composante de clarté L* que sur les autres composantes a* et b*.

Les résultats montrent que la précision de la mesure sera de l'ordre de +/- 1,5 unité en L* et de l'ordre de +/-1 unité en a* et +/- 0,5 unité en b* pour des acquisitions réalisés dans le même environnement et dans les mêmes conditions d'éclairage.

Les variabilités sur chaque composante colorimétrique L*, a* et b* ont par ailleurs été analysées pour l'ensemble des répétitions quel que soit l'environnement d'acquisition pour chaque sujet (Tableaux 4a, 4b et 4c).

**Tableau 4a : Variabilités sur du paramètre colorimétrique L* des joues (droite et gauche) sur 3 répétitions sur l'ensemble des photos acquises.**

| | | | σ (L*) | | |
|---|---|---|---|---|---|
| | Acquisition | Répétition | Min | Moyen | Max |
| Individu 1 | 17 | 3 | 0,17 | 1,48 | 7,05 |
| Individu 2 | 28 | 3 | 0,18 | 0,95 | 3,77 |
| Individu 3 | 50 | 3 | 0,12 | 1,23 | 6,99 |

**Tableau 4b : Variabilités sur du paramètre colorimétrique a* des joues (droite et gauche) sur 3 répétitions sur l'ensemble des photos acquises.**

| | | | σ (a*) | | |
|---|---|---|---|---|---|
| | Acquisition | Répétition | Min | Moyen | Max |
| Individu 1 | 17 | 3 | 0,09 | 1,05 | 3,77 |
| Individu 2 | 28 | 3 | 0,11 | 0,64 | 2,78 |
| Individu 3 | 50 | 3 | 0,04 | 0,97 | 3,83 |

**Tableau 4c : Variabilités sur du paramètre colorimétrique b* des joues (droite et gauche) sur 3 répétitions sur l'ensemble des photos acquises.**

| | | | σ (b*) | | |
|---|---|---|---|---|---|
| | Acquisition | Répétition | Min | Moyen | Max |
| Individu 1 | 17 | 3 | 0,06 | 1,31 | 6,00 |
| Individu 2 | 28 | 3 | 0,30 | 0,87 | 1,80 |
| Individu 3 | 50 | 3 | 0,07 | 1,17 | 2,95 |

On remarque que les variabilités moyennes globales sont relativement bonnes sur chaque composante. On observe néanmoins des variabilités maximales relativement importantes surtout sur la composante L* qui sont principalement dues aux variations d'illumination à chaque condition d'acquisition.

On va maintenant analyser l'évolution colorimétrique sur les joues de chacun des 3 sujets (Individus 1, 2 et 3) au cours du temps.

En premier lieu, on va analyser l'effet temps global sur 20 jours.

Cette analyse a été réalisée sur les photos prises par l'individu 1 pendant 20 jours avec le deuxième appareil Galaxy S4 mini avec un total de 51 photos.

Les données colorimétriques sont extraites sur les deux joues après recalage colorimétrique avec la mire numérique 30 patchs.

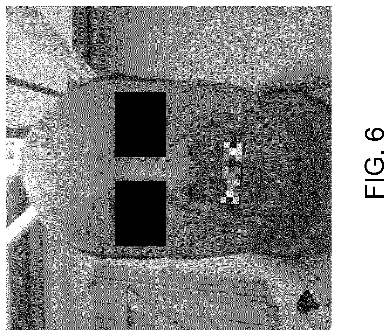

Les zones d'analyse ont été définies manuellement sur chaque photo comme le montre la figure 6.

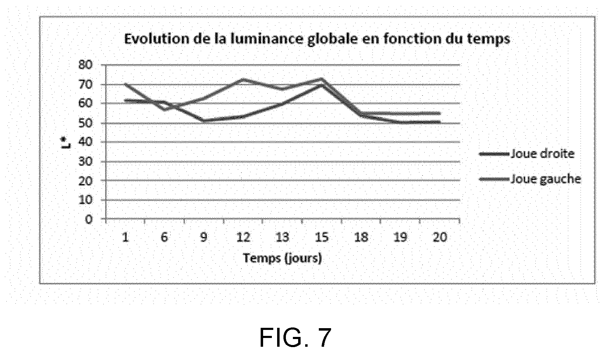

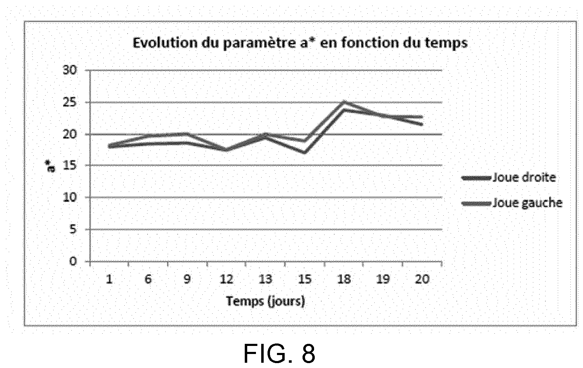

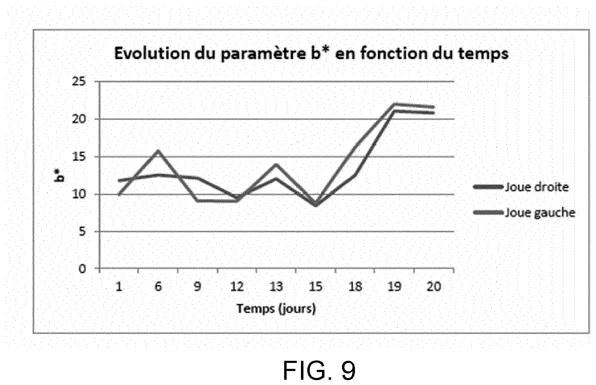

Afin d'analyser l'effet temps, on calcule la moyenne des données colorimétriques acquises à chaque journée. Cela permet notamment d'optimiser le rapport signal sur bruit. Les figures 7, 8 et 9 montrent l'évolution de respectivement des paramètres L*, a* et b* en fonction du temps sur 20 jours.

On remarque alors de forts écarts entre les joues droites et gauche en clarté (L*) notamment au 12ème jour. Cela est principalement dû à des dérives d'éclairages ou à des ombres portées sur le visage lors de la prise d'image.

Le paramètre a* (axe vert/rouge) évolue de la même façon pour les 2 joues. Le paramètre b* (bleu/jaune) présente, quant à lui, de fortes variabilités.

Si l'on étudie l'évolution générale de chaque paramètre, on constate une évolution très différente à partir du 18ème jour avec une diminution significative des paramètres L* et a* et une augmentation importante du paramètre b*.

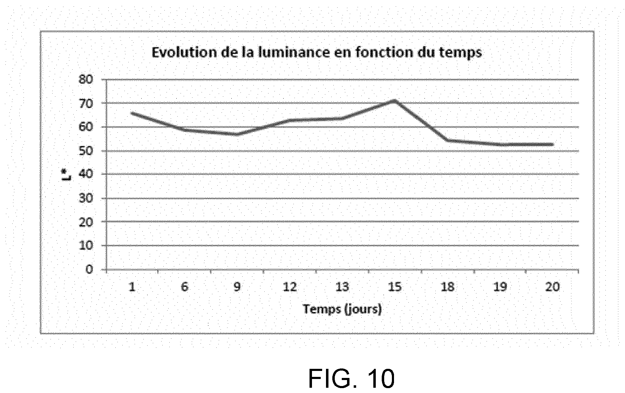

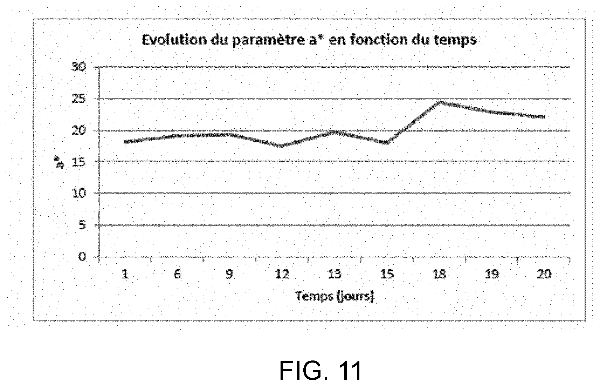

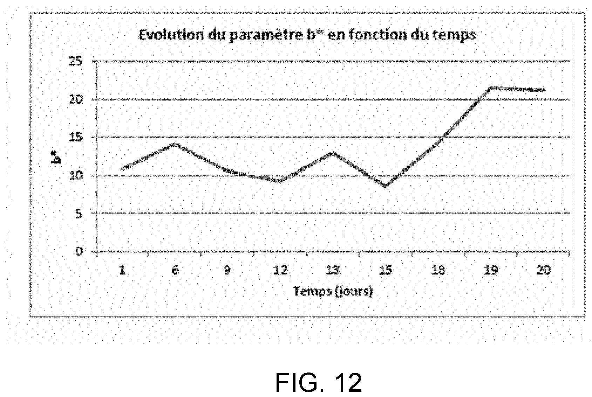

On retrouve les mêmes tendances sur les graphiques des figures 10, 11 et 12, qui présentent l'évolution de chaque paramètre colorimétrique pour la moyenne des deux joues par jour.

On va maintenant analyser l'effet temps sur 17 jours.

Cette analyse a été réalisée sur les photos prises par le deuxième individu pendant 17 jours avec le premier appareil Galaxy S4 avec un total de 84 photos.

Les données colorimétriques sont extraites sur les deux joues après recalage colorimétrique avec la mire numérique 30 patchs.

Les zones d'analyse ont été définies manuellement sur chaque photo comme pour l'analyse précédente.

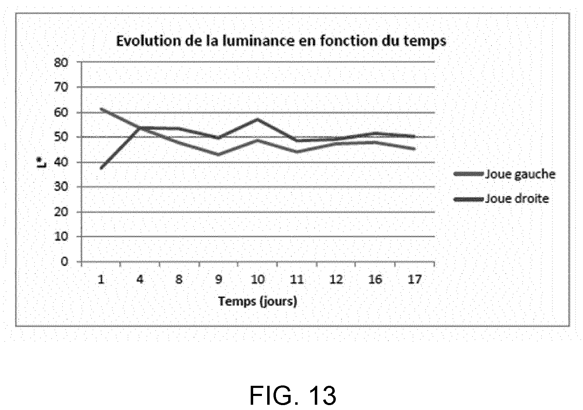

Les figures 13 à 15 montrent l'évolution de l'ensemble des paramètres colorimétriques indépendamment pour chaque joue. Les données ont été moyennées pour chaque journée.

Comme illustré sur la figure 13, on constate que le premier jour, les 2 joues sont très différemment exposées avec des valeurs de luminosité très différentes (20 unités d'écarts). Pour les autres temps d'analyse, la joue droite est toujours plus exposée que la joue gauche mais dans des proportions beaucoup moins importantes (5 à 10 unités d'écart).

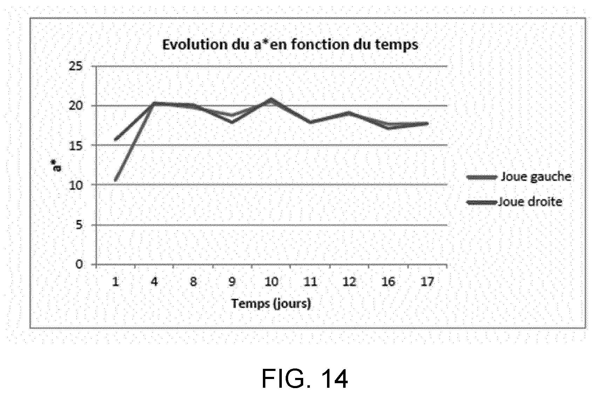

Le paramètre a* (axe rouge/vert), illustré sur la figure 14, semble identique entre les 2 joues au cours du temps sauf au premier temps.

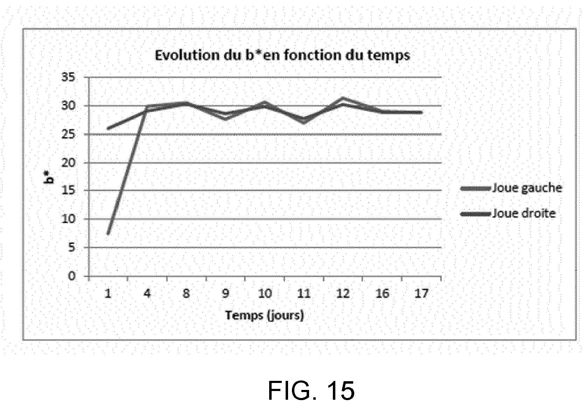

Comme pour le paramètre a*, le paramètre b* (axe jaune/bleu), illustré sur la figure 15, est relativement identique quel que soit le côté du visage analysé sauf pour le premier temps ou les différences gauche/droite sont très importantes.

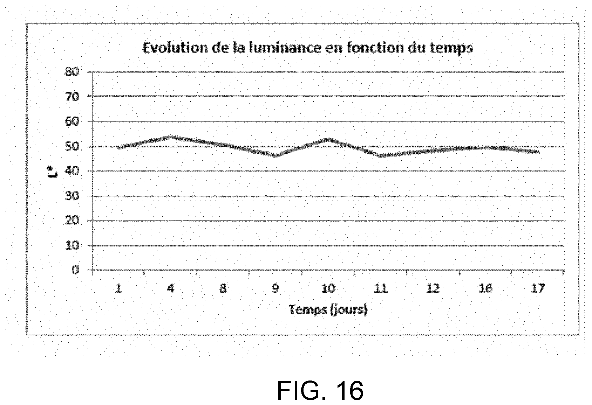

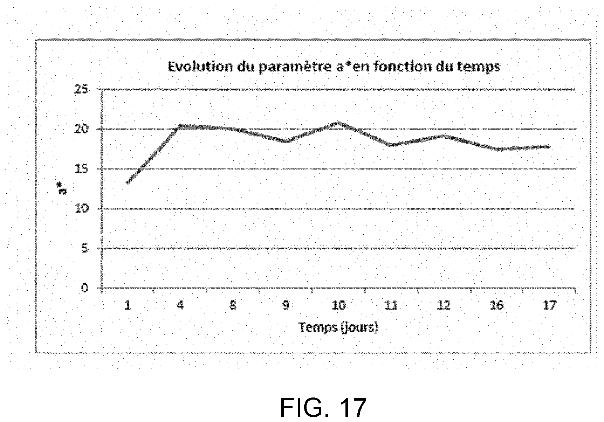

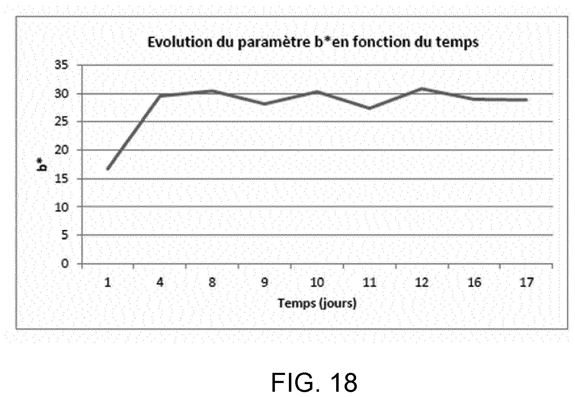

On va maintenant étudier l'évolution colorimétrique globale du visage (joue droite et joue gauche confondues), illustrée sur les figures 16 à 18.

Si l'on considère que le premier temps correspondant à un problème d'acquisition (liés à un éclairage inhomogène sur les 2 joues), on observe une quasi stabilité des différents paramètres colorimétriques L*, a* et b* au cours du temps avec des différences de seulement quelques unités.

Ces résultats montrent que la peau du deuxième individu n'a pas évoluée colorimétriquement pendant les 17 jours d'analyse.

On va maintenant analyser l'effet bronzage sur les mesures.

Cette analyse a été réalisée sur les photos prises par le deuxième individu pendant 14 jours avec le premier appareil Galaxy S4 avec un total de 150 photos.

Les données colorimétriques sont extraites sur les deux joues après recalage colorimétrique avec la mire numérique 30 patchs.

Les zones d'analyse ont été définies manuellement sur chaque photo.

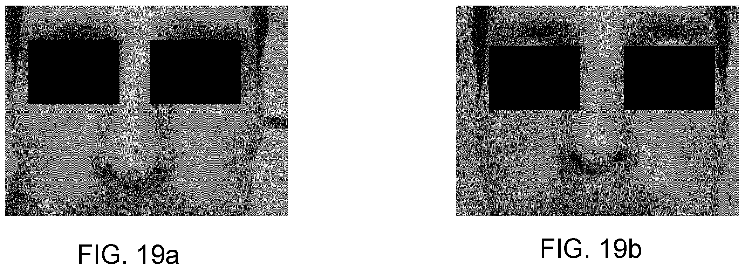

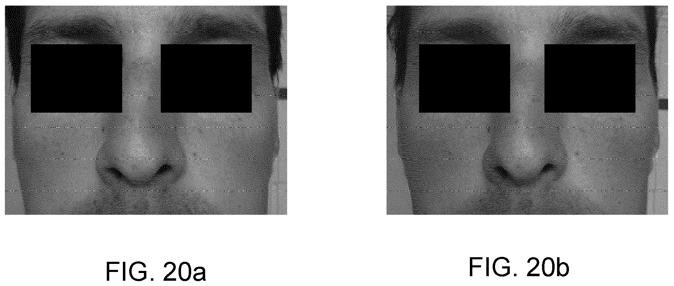

L'évolution de la couleur de la peau est visible sur les photos acquises comme le montrent les figures 19a, 19b, 20a et 20b.

On va, dans un premier temps, s'intéresser à l'évolution colorimétrique sur les 2 joues dans le même environnement lumineux. Les photos ont été acquises devant un plafonnier à tube fluorescent dans une pièce fermée à la même heure tous les jours pendant 12 jours.

L'orientation du visage par rapport au plafonnier lumineux n'a pas été contrôlée afin d'étudier les variabilités de mesure en fonction de la position du visage par rapport à l'illumination.

Trois photos ont été acquises chaque jour et les graphiques suivants montrent les résultats obtenus pendant 12 jours d'analyse.

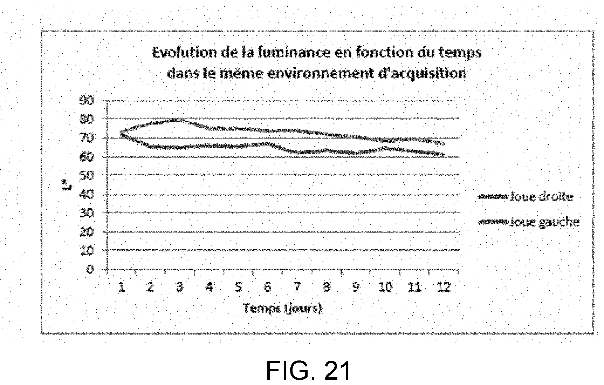

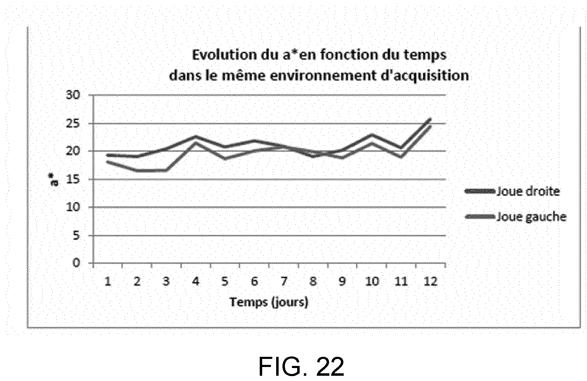

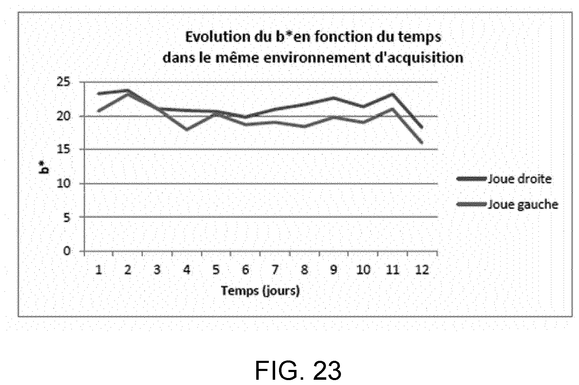

A travers les figures 21, 22 et 23, on constate que la joue gauche est globalement toujours plus surexposée que la joue droite mais que les évolutions sont similaires pour les 3 paramètres.

L'évolution globale de la peau sur les 2 joues en même temps et pour toutes les conditions d'éclairages et toutes les prises de vue acquises par jour va maintenant être examinée. Chaque jour, on effectue la moyenne de l'ensemble des données relatives aux photos acquises le matin, le midi et le soir dans des environnements lumineux différents. Les graphiques suivants montrent alors l'évolution des paramètres colorimétriques L*, a* et b* pour chaque jour.

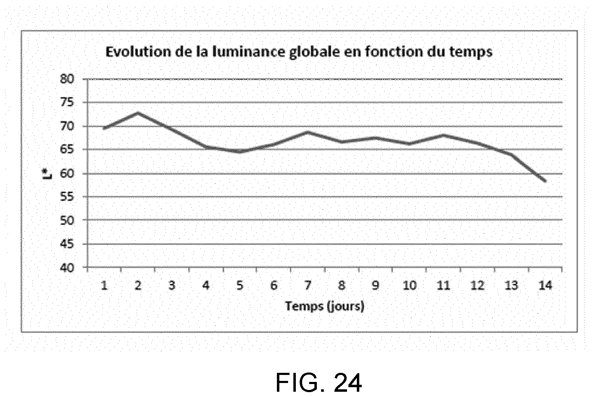

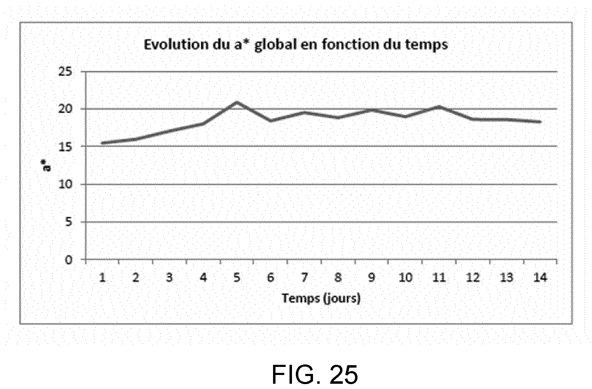

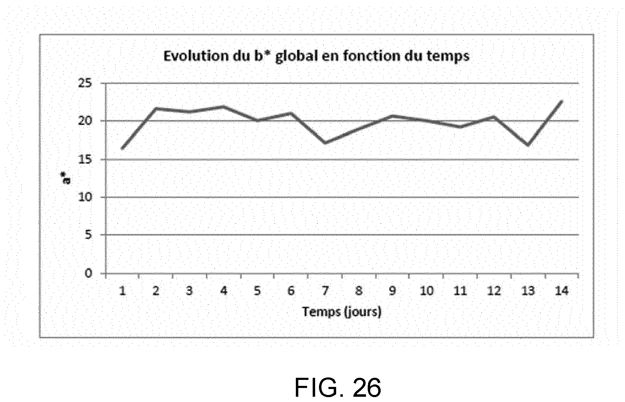

Sur les figures 24, 25 et 26, on constate que le paramètre de clarté L* diminue de plus de 10 unités en 12 jours en moyennant l'ensemble des mesures effectuées chaque jour soit un minimum de 9 photos par jour dans 3 conditions d'éclairage différentes à chaque fois. Cela montre que la peau s'est assombrie et présente donc une évolution du bronzage au cours du temps.

Les évolutions plus importantes entre le 2ème et le 3ème jour et entre le 13ème et le 14ème jour correspondent bien à des fortes expositions au soleil ces journées-là.

De plus le paramètre de rougeur (a*) augmente de plus de 4 unités sur les 12 jours d'analyse ce qui montre un rougissement de la peau. Le paramètre b* reste, quant à lui, quasiment constant tout au long de l'étude.

Comme indiqué précédemment, le logiciel d'acquisition de données « SmartCam » permet d'enregistrer des données relatives aux différents capteurs pour chaque acquisition. On va maintenant étudier l'influence des données de ces différents capteurs sur l'acquisition des images et les résultats obtenus précédemment, et tout d'abord analyser les différents capteurs sur un période de 12 jours

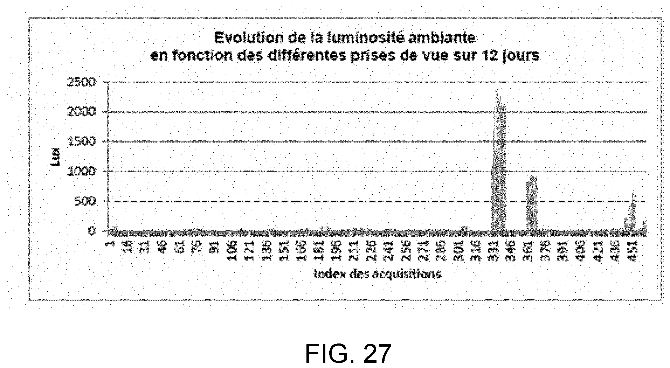

Le graphique de la figure 27 présente les données acquises par le troisième individu pendant 12 jours avec un le premier appareil, le téléphone Samsung Galaxy S4.

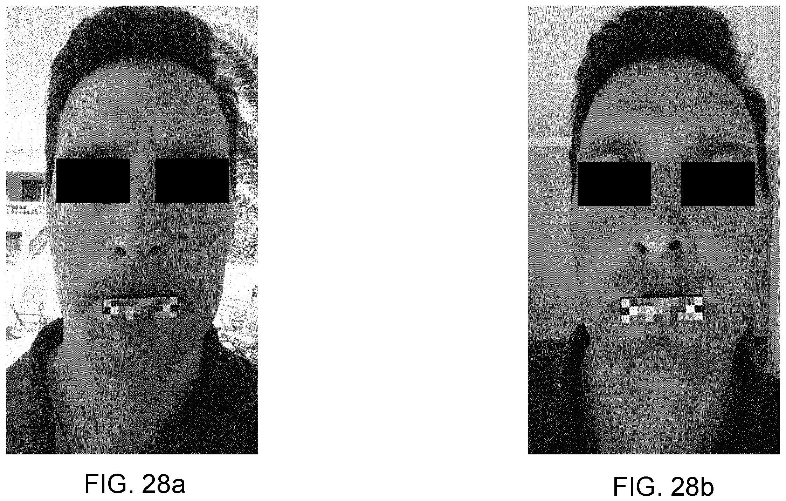

Les figures 28a et 28b montrent des photos avec un niveau de luminosité ambiante très important, respectivement 2094 lux et 912 lux.

On va maintenant étudier les autres données en fonction du temps.

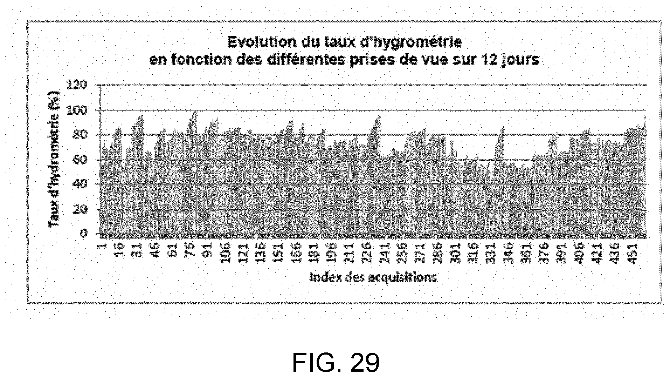

La figure 29 illustre l'évolution du taux d'hygrométrie au cours du temps. Le taux d'hygrométrie est très variable en fonction du lieu de la prise d'image. Dans les pièces d'eau (salle de bain par exemple) le taux d'humidité peut être très élevé. Aucune corrélation entre le taux d'humidité ambiant et le brillant (L*) sur les photos acquises n'a été identifiée.

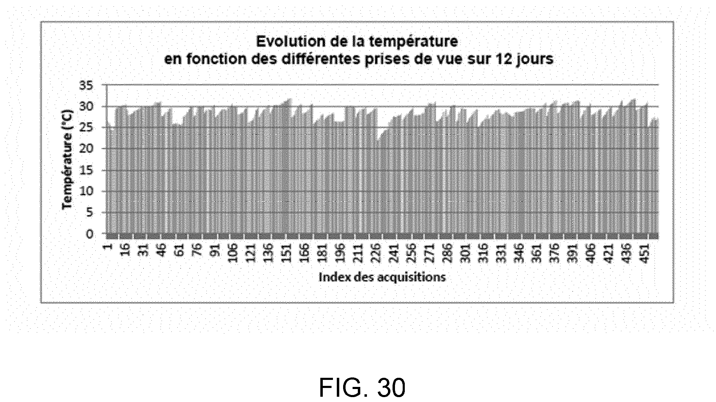

La figure 30 illustre l'évolution de la température au cours du temps. La température ambiante peut être un facteur important pour la sensation de l'effet de chaleur lors de l'effet flush. Cette donnée pourrait être utilisée en complément de la chaleur ressentie. Par contre, dans le cadre d'une étude de faisabilité, aucune corrélation entre la température et l'évolution des paramètres colorimétriques n'a été constatée, la température étant restée relativement stable au cours de l'étude (entre 23° et 32°C).

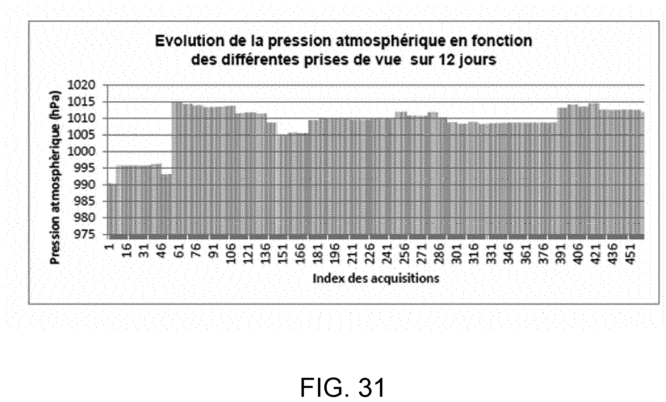

La figure 31 illustre l'évolution de la pression atmosphérique au cours du temps. La pression atmosphérique montre notamment un changement de lieu au début de l'étude mais ne permet pas une corrélation avec les valeurs colorimétriques mesurées.

Les différents capteurs vont maintenant être analysés sur une période de 15 jours

Les données ont été acquises par le deuxième individu pendant 15 jours avec le premier appareil, un Samsung Galaxy S4.

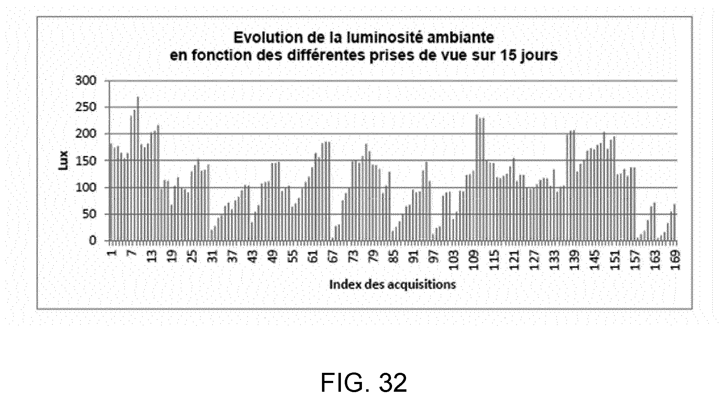

Les différences de luminosité ambiante observées sur les acquisitions de photos, illustrées sur la figure 32, ne sont pas source de dérive lors de l'acquisition. Les variations de 260 lux maximum restent très faibles.

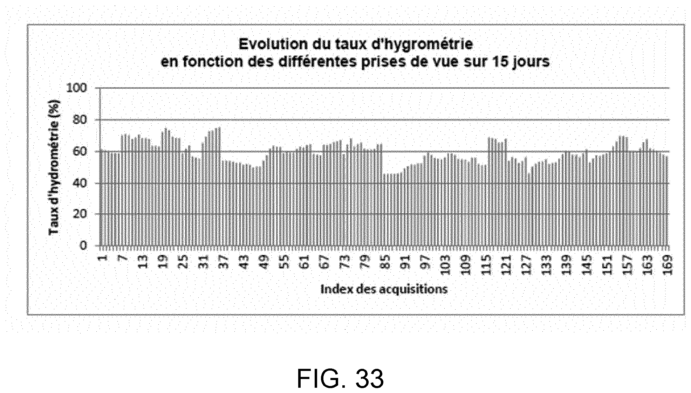

Le taux d'hygrométrie, illustré sur la figure 33, varie en fonction de temps avec une moyenne globale de 54%. Aucune corrélation entre ces valeurs et l'évolution des paramètres colorimétriques n'a été identifiée.

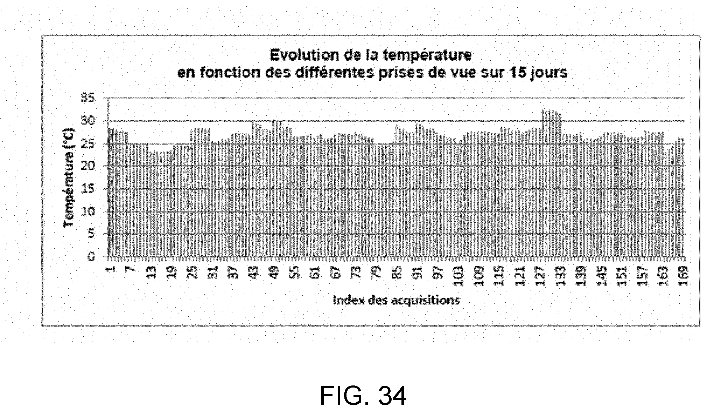

La température, illustrée sur la figure 34, est, elle aussi, relativement stable et pourrait présenter une donnée intéressante pour l'étude clinique. Elle n'influe pas, dans cette étude, significativement sur la couleur de la peau des joues.

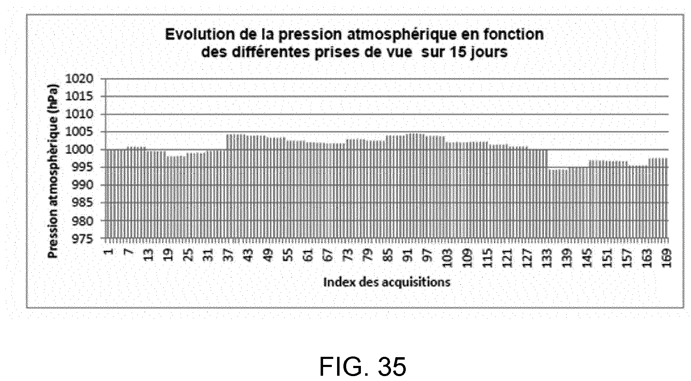

Là encore, la pression atmosphérique, illustrée sur la figure 35, varie légèrement mais n'est pas un indicateur qui peut être corrélé avec la couleur de la peau.

Les données ont été acquises par le premier individu pendant 20 jours avec le deuxième appareil, un Samsung® Galaxy S4 Mini®. Cet appareil possède uniquement un gyroscope et un capteur de luminosité.

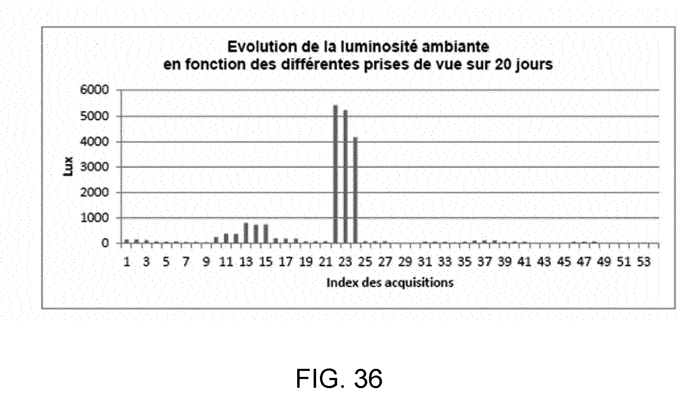

On s'intéressera ici uniquement aux données relatives à la luminosité ambiante comme le présente le graphique de la figure 36.

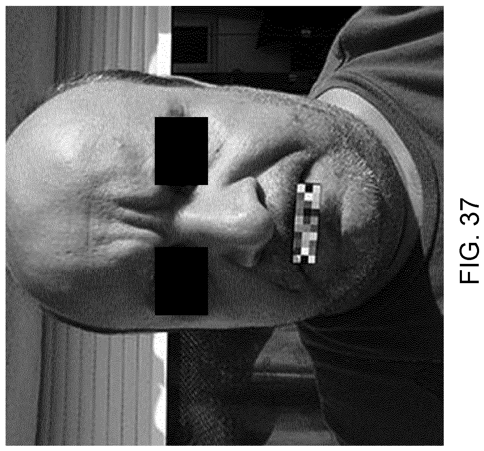

On remarque alors que certaines photos ont été acquises avec une luminosité ambiante dépassant les 4000 lux. La figure 37 suivante présente une photo relative à ces acquisitions.

L'inclinaison de l'appareil va maintenant être étudiée.

Lors de chaque acquisition, les informations concernant l'inclinaison de l'appareil sont stockées dans la base de données associée avec le logiciel d'acquisition « SmartCam ».

Après analyse de l'ensemble des données d'inclinaison de l'appareil à chaque acquisition, reportées dans le tableau 5 suivant, on constate que la valeur médiane est relativement constante entre les sujets. Cependant les angles d'acquisition peuvent varier de façon relativement importante avec un écart maximum de 30°.

**Tableau 5 : Angles d'inclinaison des prises de vues.**

| | Min | Médian | Max |
|---|---|---|---|
| Individu 1 | 73,36° | 85,05° | 105,88° |
| Individu 2 | 73,43° | 87,38° | 100,57° |
| Individu 3 | 66,04° | 84,78° | 96,80° |

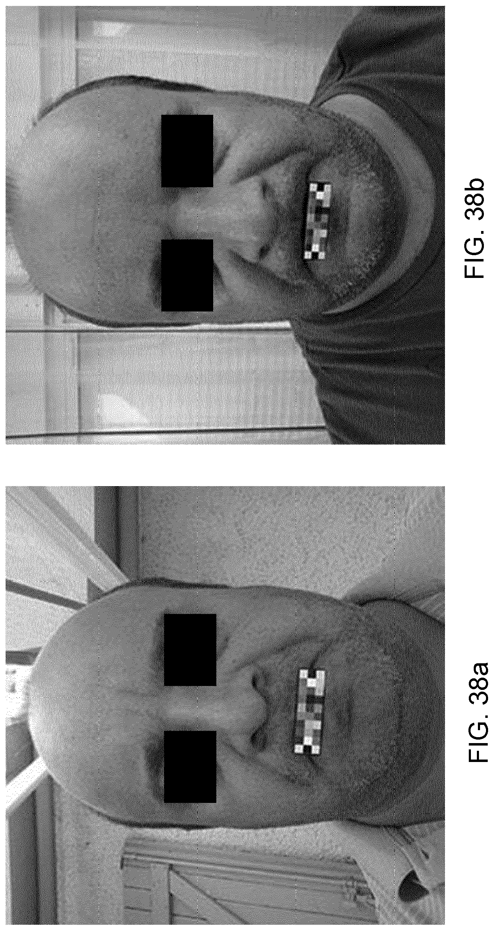

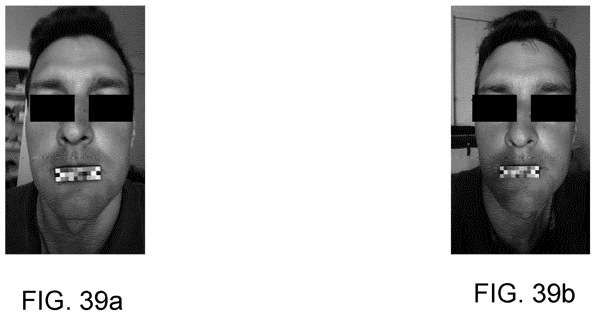

Ces variations de l'angle d'inclinaison peuvent modifier la zone d'analyse sur les joues mais également la géométrie d'éclairage par rapport à l'axe de prise de vue comme le montrent les figures 38a, 38b, 39a et 39b. La photographie de la figure 38a est prise avec une inclinaison verticale de l'appareil à 73° tandis que celle de la figure 38b est acquise avec une inclinaison verticale de 105°. La photographie de la figure 39a est prise avec une inclinaison verticale de l'appareil à 66° tandis que celle de la figure 39b est acquise avec une inclinaison verticale de 96°.

Afin de contrôler au mieux l'orientation de la prise de vue, il sera nécessaire d'activer l'indicateur d'inclinaison (figure 2c) via la partie administration du logiciel permettant de contraindre l'angle d'acquisition, entre 85° et 95° par exemple, au moment de la prise d'image pour limiter les variabilités liées à l'angle d'acquisition.

Au vu de ce qui précède, les deux systèmes d'acquisitions de type fournissent des images de bonne qualité pour une analyse colorimétrique globale. La résolution des images est satisfaisante avec un champ rectangulaire pour le S4 et carré pour le S4 Mini qui limite donc la résolution si l'on veut avoir le visage complet dans le champ.

On privilégiera donc le premier appareil qui possède également un écran plus grand pour la visualisation temps réel au moment de l'acquisition.

On notera cependant un effet relativement important de la compression JPEG sur les photos résultantes lors des forts grandissements. Ces systèmes ne donnent pas accès aux données brutes (format RAW) des photos en sortie de capteur et imposent le format JPEG avec faible compression.

En ce qui concerne l'application logicielle, celle-ci est relativement intuitive et présente l'ensemble des fonctionnalités désirées.

Avantageusement, un contrôle de la luminosité ambiante par l'intermédiaire d'un capteur de luminosité intégré dans un téléphone intelligent pour éviter la surexposition d'une zone particulière sur le visage ou des conditions d'éclairage trop éloignées des conditions initiales est mis en place. Un message apparaitra sur l'écran pour avertir des utilisateurs si la condition de luminosité n'est pas satisfaisante.

Il devra cependant évoluer pour intégrer d'autres fonctionnalités relatives à l'étude clinique.

Plusieurs optimisations sont, d'ores et déjà, envisagées :
- ajout d'un contrôle de la luminosité ambiante pour éviter la surexposition d'une zone particulière sur le visage ou des conditions d'éclairage trop éloignées des conditions initiales,
- ajout d'un contrôle de l'image acquise pour valider la netteté et ainsi limiter le flou de bouger,
- ajout d'une validation de la cohérence colorimétrique entre différentes zones du visage (2 joues et front) pour essayer de limiter au mieux les dérives d'éclairement,
- ajout d'une ou plusieurs zones de référence colorimétrique sur le visage à T0 pour essayer de limiter les dérives d'une acquisition à l'autre, et
- mise en place d'un écran blanc avec une luminosité maximale lors de la prise d'image pour essayer d'obtenir un éclairage relativement important provenant de l'appareil.

Par ailleurs, les 3 mires conviennent pour l'étape de recalage colorimétrique des images. Cependant la mire numérique semble la plus appropriée car elle permet d'ajuster non seulement la température de couleur, comme avec la mire grise, mais aussi d'optimiser certaines teintes spécifiques.

La mire Munsell, bien que très diffuse, n'est pas plus performante que la mire réalisée en impression numérique étant donné les grandes variations d'éclairage.

Le support de mire réalisé pour cette étude semble convenir pour un bon repositionnement devant le sujet. Ses dimensions pourront être optimisées si besoin.

Le principal problème pour le recalage colorimétrique est que les variations colorimétriques sur la mire ne tiennent pas en compte l'ensemble des variations sur le visage à cause des différences trop importantes de géométrie d'éclairage. C'est pour cela qu'il faut essayer de contraindre au mieux l'environnement lumineux lors de la prise d'image. L'idéal serait d'avoir un éclairage uniforme très diffus.

On va maintenant procéder à l'analyse des données colorimétriques.

La mire colorimétrique permet d'optimiser de près de 50% les dérives colorimétriques entre chaque prise de vue.

Après recalage des images, la répétabilité du système est de l'ordre de 1,5 unité en L* et environ 1 unité en a* et b* dans des conditions d'éclairement uniformes.

Dans des conditions d'éclairage non uniformes sur la zone du visage, la variation de géométrie d'une prise d'image à une autre peut entraîner des écarts de 7 unités en L* et b* et de 4 unités en a* au maximum. Ces variations sont d'autant plus grandes si l'on change de lieu et de type d'éclairage.

Cependant, il est possible en moyennant l'ensemble des données d'obtenir des résultats intéressants sur l'évolution colorimétrique de la peau comme l'effet du bronzage au cours du temps.

Dans le cadre de l'analyse de l'effet flush, il sera donc nécessaire de multiplier les acquisitions pour optimiser le rapport signal/bruit.

Plusieurs solutions sont à explorer :
- utiliser le magnétomètre pour obliger l'utilisateur à changer d'orientation et donc changer la géométrie d'éclairage,
- utiliser des paramètres colorimétriques spécifiques comme le dH (écart de tonalité chromatique) qui ne tient pas compte de la clarté L*,
- utiliser des paramètres invariants en fonction des ombres comme l'espace RGB normalisé ou des espaces spécifiques de type L1L2L3,
- utiliser le paramètre a* qui semble moins influencé par l'environnement lumineux et qui tient compte des rougeurs, et
- utiliser des zones de référence sur la peau comme le menton ou le front pour effectuer un calcul de contraste entre la peau « saine » et la peau « avec effet flush ».

En outre, les données colorimétriques peuvent être analysées avantageusement pour une auto-évaluation des maladies de la peau tels que mélasma, nævus, rosacée, érythème, rougissement, acné, psoriasis, dermatites, kératose actinique, éruption cutanée et dermite séborrhéique.

Il est par ailleurs possible d'utiliser divers types de capteurs prévus dans les appareils de prise de vues.

Les capteurs de température, de pression et d'hygrométrie présents dans le premier téléphone permettent d'extraire des informations intéressantes mais n'aident pas forcément à stabiliser la prise d'image ou à contrôler l'environnement d'acquisition.

Par contre les capteurs de luminosité et d'inclinaison (gyroscope), qui sont également présents dans le deuxième appareil, peuvent aider à contraindre la prise d'image.

Ainsi, les divers capteurs des appareils de prise de vues sont utilisés pour acquérir des paramètres pourtant notamment sur les conditions de prise de vues. L'application logicielle d'acquisition « SmartCam » est alors programmée pour corriger les composants colorimétriques de l'effet des paramètres mesurés sur le visage.

En d'autres termes, il sera possible d'intégrer une prévisualisation temps réel de ces capteurs au moment de la prise d'image pour contraindre l'utilisateur à positionner correctement son appareil ou à changer de lieu car l'ambiance lumineuse est beaucoup trop importante (supérieure à 500 lux par exemple).

Afin d'optimiser la prise d'image en très haute résolution, d'autres objectifs, tels que ceux commercialisés sous la marque Sony® peuvent être utilisés. Ces objectifs connectés sur smartphone se connectent en Wi-Fi à un smartphone Android ou un iPhone® et permettent d'effectuer des prises de vue d'excellente qualité. Actuellement, le format JPG est le seul pris en charge, le RAW arrivera plus tard.

L'objectif QX10 est composé d'un capteur Exmor R CMOS de 18,2 mégapixels auquel vient s'ajouter un zoom x 3,6 Carl Zeiss®. Une optique offrant une ouverture comprise entre 3,5 et 3,9 avec un microphone et un bouton dédié à l'obturateur. Le QX100 est composé d'un capteur CMOS de 20,2 mégapixels, un zoom optique 3,6x f/1,8, un microphone, un bouton dédié à l'obturateur et deux bagues pour les réglages manuels.

Les objectifs s'adaptent sur le smartphone et une application Sony® spécifique permet de prendre des clichés. Le smartphone sert donc à piloter l'objectif photo. Il faudra savoir s'il est possible de piloter ces objectifs avec une application externe. Leur prix est compris entre 250 et 450 dollars américains.

Au niveau des diverses fonctionnalités de ces objectifs Sony, on peut retrouver divers modes de fixation, des connexions Wi-Fi et NFC, une compatibilité Android et iOS, l'affichage direct des images sur l'écran du terminal connecté, la sauvegarde simultanée sur l'objectif et le smartphone connecté, les contrôles manuels depuis le smartphone ce qui permet de piloter les objectifs directement depuis le terminal connecté, l'enregistrement de vidéos en Full HD 1080p.

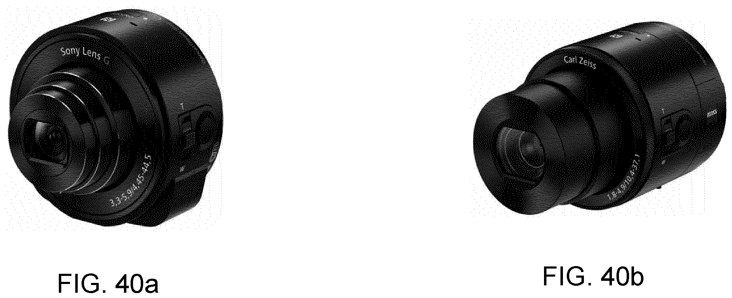

Le Sony QX10, illustré sur la figure 40a, est construit autour d'un capteur photo CMOS Exmor de 1/2,3 " d'une définition de 18 millions de pixels. Son zoom 10x stabilisé couvre la plage focale 25-250 mm avec des ouvertures de f/3,3-5,9. Pour le traitement de l'image, il bénéficie du processeur BIONZ. La sensibilité s'étend de 100 à 12800 ISO en fonction des modes de prise de vues. Ses dimensions sont de 6,24 x 6,18 x 3,33 cm pour un poids de 90 grammes.

Le Sony® QX100, illustré sur la figure 40b, est construit autour d'un capteur photo CMOS Exmor de 1" avec une définition de 20 millions de pixels et une optique (Carl Zeiss®) stabilisée x 3,6 couvrant la plage focale 28-100 mm. Ses ouvertures se situent à f/1,8-4,9. Le QX100 bénéficie aussi du processeur de traitement d'images BIONZ. Sa sensibilité s'étend de 160 à 25600 ISO. Ses dimensions sont de 6,25 x 6,25 x 5,55 cm pour 179 grammes.

On va enfin décrire en référence aux figures 41a, 41b, 42a, 42b et 43 une utilisation pratique de l'application logicielle embarquée dans les systèmes de prise de vues dans le cadre d'un traitement d'un flush par le médicament Brimonidine.

Afin de tester l'application logicielle embarquée dans les téléphones, un essai clinique portant sur un traitement de flush par Brimonidine a été réalisé, au cours duquel des prises de vues ont été faites en utilisant le logiciel d'acquisition « SmartCam ».

Cette étude en phase IIa, généralement conçue pour évaluer des besoins de dosage, comporte deux périodes. Pour tenir compte des variations entre individus, cette étude a été conçue pour améliorer la puissance statistique dans la première période et un plan d'étude a été prévu dans la deuxième période. Avantageusement, il s'agit une étude randomisée en double aveugle avec répartition aléatoire et placebo contrôlé.

La première période est effectuée dans un centre de traitement et comporte trois séances. Elle dure une semaine.

La deuxième période dure quatre semaines et est réalisée par un sujet à demeure. Elle sert à valider le modèle de rougissement et identifier des problèmes ou difficultés.

24 sujets avec ETR (Erythematotelangiectatic rosacea) et 12 sujets avec PPR (Papulopustular rosacea) ont participé à cette étude.

L'étude complète dure approximativement 13 semaines. Il y a une participation subjective de 5 semaines et une période de dépistage jusqu'à 4 semaines pour chaque sujet.

La première période de l'étude basée sur le modèle de flush (rougissement) est effectuée dans un centre de traitement. Cette période vise à réaliser une évaluation objective de la variabilité intra-individuelle sur chaque sujet.

Dans cette première semaine de l'étude, on met en place des tests sur des demi-visages de chaque sujet avec deux traitements différents : Véhicule (équivalant de placebo, non actif) et Brimonidine 0,5% (produit à tester, actif).

Comme illustré sur la figure 41a, le véhicule est appliqué sur les deux demi-visages du sujet dans la première journée. Deux jours après, le un traitement à base de Brimonidine 0,5% est appliqué sur un des deux demi-visages du sujet. Dans la cinquième journée, tous les véhicules sont remplacés par le Brimonidine 0,5%.

La deuxième période de l'étude est réalisée à domicile par chaque sujet par l'intermédiaire d'un téléphone intelligent. Il s'agit une évaluation subjective basée sur des essais en deux groupes parallèles (figure 41b).

La deuxième période dure 4 semaines. Un premier groupe de sujets met en oeuvre un traitement à base de Brimonidine sur le visage complet et le placebo est appliqué pour un deuxième groupe de sujets pendant les deux premières semaines. Dans les deux semaines suivantes, les deux groupes des sujets échangent leurs produits à rester.

Pendant la deuxième période de l'étude, chaque sujet utilise le premier téléphone intelligent et le logiciel d'acquisition de données « SmartCam ».

Comme indiqué précédemment, l'application logicielle permet le repositionnement rapide du sujet à l'aide des deux axes horizontaux et d'un axe vertical et le contrôle de verticalité d'acquisition d'images à l'aide du capteur gyroscope du téléphone intelligent.

Les procédés d'acquisition sont identiques à ceux présentés ci-avant. La mire numérique est adoptée dans cette période de l'étude. Chaque sujet doit prendre une première photo de référence le matin (au lever) et une photo supplémentaire lors de chaque effet flush pendant la journée.

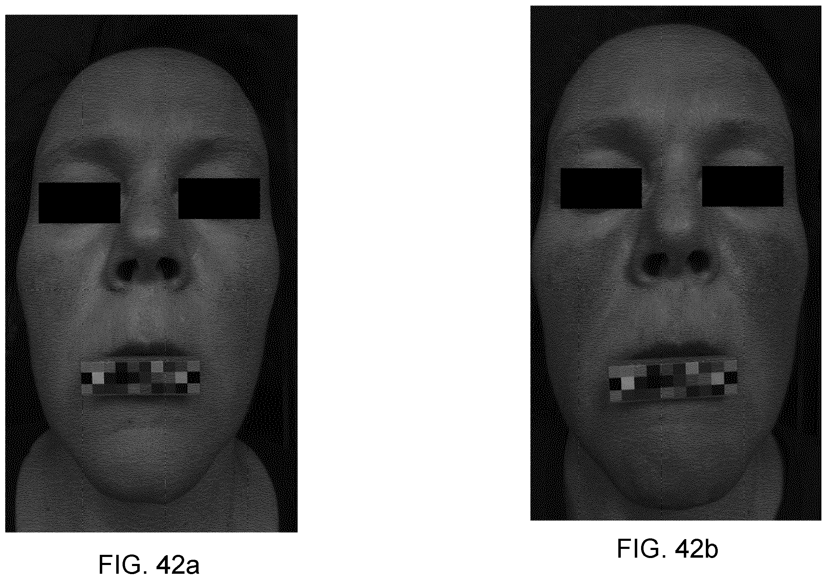

Les figures 42a et 42b montrent un exemple d'un sujet dans le onzième jour de la deuxième période. La photo visible sur la figure 42a est prise dans la matinée à 08h20 et considérée comme référence. Lors d'un effet flush à 15h59, une photo supplémentaire est prise (figure 42b). Les joues (gauche et droite) du sujet sur la figure 42b sont notablement beaucoup plus rouges que celles montrées sur la photo du matin (figure 42a).

Les résultats de l'étude lors de la deuxième période sur le changement du paramètre colorimétrique a* depuis le matin sont montrés dans le tableau 6. Les abréviations PP et ITT dans le tableau 6 correspondent respectivement aux analyses « Per Protocol » et « Intention-To-Treat » connues par l'homme du métier.

On peut constater que les moyens et les médians du changement a* moyen depuis le matin sont toujours positifs. En d'autres termes, les joues lors d'effets flushs sont plus rouges que le matin. Le fait que l'intervalle de confiance 95% se situe entre [0,47, 2,07] confirme la capacité de l'application logicielle à détecter les flushs.

**Tableau 6 : Changement du a* moyen depuis le matin.**

| Changement a* moyen depuis le matin | | Brimonidine 0,5% | Placebo | Total |
|---|---|---|---|---|
| Deux premières semaines/ PP | Nombre | 14 | 16 | 30 |
| | Moyen ± σ | 1,15±3,24 | 1,34±2,10 | 1,25±2,64 |
| | Médian | 0,92 | 1,04 | 0,93 |
| | (Min, Max) | (-4,3, 10,2) | (-1,4, 5,9) | (-4,3, 10,2) |
| Deux première semaines/ ITT | Nombre | 15 | 16 | 31 |
| | Moyen ± σ | 1,44±3,32 | 1,34±2,10 | 1,39±2,71 |
| | Médian | 0,94 | 1,04 | 0,94 |
| | (Min, Max) | (-4,3, 10,2) | (-1,4, 5,9) | (-4,3, 10,2) |
| Deux dernières semaines/ PP | Nombre | 15 | 14 | 29 |
| | Moyen ± σ | 1,47±2,91 | 1,2±2,51 | 1,34±2,68 |

| | Médian | 1,98 | 1,58 | 1,67 |
|---|---|---|---|---|
| | (Min, Max) | (-4,1, 5,5) | (-2,9, 5,3) | (-4,1, 5,5) |
| Deux dernières semaines/ ITT | Nombre | 15 | 14 | 29 |
| | Moyen ± σ | 1,47±2,91 | 1,2±2,51 | 1,34±2,68 |
| | Médian | 1,98 | 1,58 | 1,67 |
| | (Min, Max) | (-4,1, 5,5) | (-2,9, 5,3) | (-4,1, 5,5) |
| Total/PP | Nombre | 29 | 30 | 59 |
| | Moyen ± σ | 1,32±3,02 | 1,34±2,10 | 1,25±2,64 |
| | Médian | 1,49 | 1,26 | 1,31 |
| | (Min, Max) | (-4,3, 10,2) | (-2,9, 5,9) | (-4,3, 10,2) |
| Total/ITT | Nombre | 30 | 30 | 60 |
| | Moyen ± σ | 1,46±3,074 | 1,27±2,26 | 1,37±2,67 |
| | Médian | 1,58 | 1,26 | 1,40 |
| | (Min, Max) | (-4,3, 10,2) | (-2,9, 5,9) | (-4,3, 10,2) |

Par ailleurs, l'application logicielle permet également d'étudier la distribution des flushs pendant la journée grâce aux données de temps (date et heure) stockées, relatives à chaque prise d'images.

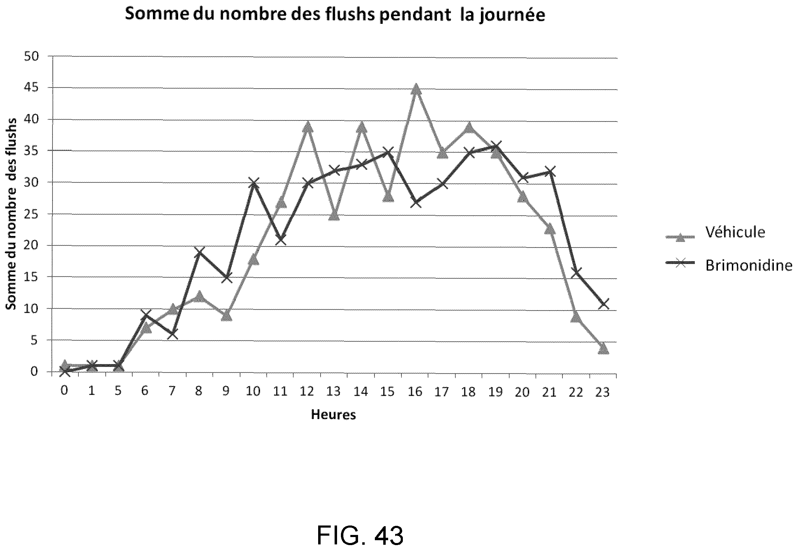

Comme illustré sur la figure 43, on peut observer la somme du nombre des flushs distribués tout au long de la journée. Il y a plus de flushs apparus dans l'après-midi (12h-19h) que dans le reste du temps de la journée. L'application d'un traitement permet de diminuer l'apparition des flushs dans cette période de la journée. Ces données importantes peuvent être ensuite analysées afin de mettre fin à un traitement.

Avec la capacité de détecter des flushs et enregistrer leurs distributions pendant la journée, l'application rend ainsi possible de réaliser des auto-évaluations de maladies de peau tels que le mélasma, le nævus, la rosacée, l'érythème, le rougissement, l'acné, le psoriasis, la dermatite, la kératose actinique, l'éruption cutanée et la dermite séborrhéique.

## Revendications

1. Procédé d'analyse d'images du visage d'un être humain comprenant les étapes suivantes :
- on réalise l'acquisition d'au moins une image du visage de sorte que le nez, les yeux et les joues soient alignés sur des repères préexistants,
- on assure la verticalité de l'acquisition d'images par l'intermédiaire d'un indicateur d'inclinaison affichable sur un logiciel embarqué dans un système de prise de vues de façon à forcer le bon positionnement du système à la verticale au moment de la prise d'image,
- on détermine les composantes de l'image dans au moins une zone du visage,
- on compare les composantes dont une composante colorimétrique associée à la couleur rouge à des valeurs mémorisées, et
- on détermine la présence d'un effet flush si ladite composante colorimétrique est supérieure la valeur mémorisée associée à la couleur rouge.

2. Procédé selon la revendication 1, dans lequel les zones du visage sont les joues.

3. Procédé selon l'une quelconque des revendications précédentes, dans lequel les composantes sont les composantes colorimétriques L*, a*, b*.

4. Procédé selon l'une quelconque des revendications précédentes, dans lequel les valeurs mémorisées sont les valeurs des composantes colorimétriques déterminées à une date antérieure pour le même visage.

5. Procédé selon l'une quelconque des revendications précédentes, dans lequel la verticalité de l'acquisition d'images est assurée par l'intermédiaire d'un capteur interne d'un téléphone intelligent.

6. Procédé selon l'une quelconque des revendications précédentes, dans lequel on réalise l'acquisition alors que le sujet tient une mire colorimétrique par l'intermédiaire de la bouche, afin de corriger les composantes colorimétriques.

7. Procédé selon l'une quelconque des revendications précédentes, dans lequel on acquiert au moins un paramètre choisi parmi la température, la pression atmosphérique, la luminosité ambiante et l'hygrométrie, afin de corriger les composantes colorimétriques d'un effet de l'environnement sur le visage.

8. Procédé selon l'une quelconque des revendications précédentes, dans lequel les valeurs des composantes colorimétriques déterminées sont configurées pour une auto-évaluation des maladies de la peau tels que le mélasma, le naevus, la rosacée, l'érythème, le rougissement, l'acné, le psoriasis, la dermatite, la kératose actinique, l'éruption cutanée et la dermite séborrhéique.

## Patentansprüche

1. Verfahren zur Analyse von Bildern des Gesichts eines Menschen, das die folgenden Schritte enthält:
- Durchführen der Erfassung mindestens eines Bilds des Gesichts derart, dass die Nase, die Augen und die Wangen auf vorab existierenden Koordinatensystemen ausgerichtet sind;
- Gewährleisten der Vertikalität der Bilderfassung mittels eines Neigungsanzeigers, der auf einer eigenen Software eines Bildaufnahmesystems angezeigt werden kann, um die korrekte Positionierung des Systems zur Vertikalen zum Zeitpunkt der Bildaufnahme zu erzwingen,
- Bestimmen der Komponenten des Bilds in mindestens einer Zone des Gesichts,
- Vergleichen der Komponenten, darunter mindestens eine der Farbe Rot zugeordnete Farbkomponente, mit gespeicherten Werten, und
- Bestimmen des Vorhandenseins eines Flush-Effekts, wenn die Farbkomponente größer als der der Farbe Rot zugeordnete gespeicherte Wert ist.

2. Verfahren nach Anspruch 1, wobei die Zonen des Gesichts die Wangen sind.

3. Verfahren nach einem der vorhergehenden Ansprüche, wobei die Komponenten die Farbkomponenten L*, a*, b* sind.

4. Verfahren nach einem der vorhergehenden Ansprüche, wobei die gespeicherten Werte die Werte der Farbkomponenten sind, die zu einem vorhergehenden Zeitpunkt für das gleiche Gesicht bestimmt wurden.

5. Verfahren nach einem der vorhergehenden Ansprüche, wobei die Vertikalität der Bilderfassung von einem inneren Sensor eines intelligenten Telefons gewährleistet wird.

6. Verfahren nach einem der vorhergehenden Ansprüche, wobei die Erfassung durchgeführt wird, während die Person eine Farbtesttafel mittels des Munds hält, um die Farbkomponenten zu korrigieren.

7. Verfahren nach einem der vorhergehenden Ansprüche, wobei mindestens ein Parameter erfasst wird, der ausgewählt wird aus der Temperatur, dem Luftdruck, der Umgebungshelligkeit und der Feuchtigkeitsmessung, um die Farbkomponenten einer Wirkung der Umgebung auf dem Gesicht zu korrigieren.

8. Verfahren nach einem der vorhergehenden Ansprüche, wobei die Werte der bestimmten Farbkomponenten konfiguriert sind für eine Selbstbewertung von Hautkrankheiten wie Melasma, Nävus, Rosacea, Erythem, Rötung, Akne, Psoriasis, Dermatitis, aktinische Keratose, Hautausschlag und seborrhoische Dermatitis.

## Claims

1. A method for analyzing images of the face of a human being comprising the following steps:
- at least one image of the face is acquired such that the nose, the eyes and the cheeks are aligned on pre-existing markers,
- the vertical alignment of the acquisition of images is ensured via an internal sensor viewable on an embedded software in a photograph system to force the correct positioning of the device vertically at the image capture time,
- the components of the image are determined in at least one zone of the face,
- the components of which a colorimetric component associated to the color red are compared to stored values, and
- the presence of a flush effect is determined if the components are greater than the stored values.

2. The method as claimed in claim 1, in which the zones of the face are the cheeks.

3. The method as claimed in either one of the preceding claims, in which the components are the colorimetric components L*, a*, b*.

4. The method as claimed in any one of the preceding claims, in which the stored values are the values of the colorimetric components determined at an earlier date for the same face.

5. The method as claimed in any one of the preceding claims, in which the vertical alignment of the acquisition of images is ensured via an internal sensor of a smartphone.

6. The method as claimed in any one of the preceding claims, in which the acquisition is performed while the subject holds a colorimetric color chart via the mouth, in order to correct the colorimetric components.

7. The method as claimed in any one of the preceding claims, in which at least one parameter is acquired that is chosen from the temperature, the atmospheric pressure, the ambient brightness and the relative humidity, in order to correct the colorimetric components of any environmental effect on the face.

8. The method as claimed in any one of the preceding claims, in which the values of the colorimetric components determined are configured for a self-assessment of skin disorders such as melasma, nevus, rosacea, erythema, reddening, acne, psoriasis, dermatitis, actinic keratosis, rash and seborrheic dermatitis.
